# EUROPEAN PATENT APPLICATION

(11) **EP 4 512 902 A1**
(43) Date of publication of application: **26.02.2025**
(21) Application number: 23791786.9
(22) Date of filing: 13.04.2023
(51) Int. Cl.: C12N 15/87, C07K 7/08, C12N 5/00

(54) **FOREIGN SUBSTANCE INTRODUCTION CONSTRUCT AND UTILIZATION THEREOF**

(30) Priority: 20.04.2022 JP 2022069546
(71) Applicant: Toagosei Co., Ltd., Minato-ku Tokyo 105-8419 (JP)
(72) Inventor: BAILEYKOBAYASHI, Nahoko, Tokyo, 105-8419 (JP); YOSHIDA, Tetsuhiko, Tokyo, 105-8419 (JP)
(74) Representative: Bals & Vogel Patentanwälte PartGmbB
(86) International application number: PCT/JP2023/015027
(87) International publication number: WO 2023/204138

(57) **Abstract**

The present disclosure provides a technique for efficiently transferring a target foreign substance from outside a eukaryotic cell into at least a cytoplasm of the cell. A method for transferring a target foreign substance from outside a eukaryotic cell into at least a cytoplasm of the cell disclosed herein includes the steps of: (1) preparing a construct for transferring a foreign substance, the construct including a carrier peptide fragment including an amino acid sequence: RSRKYTSWYVALKRWPC (SEQ ID No.: 1), and the target foreign substance that is bonded to an N-terminus and/or a C-terminus of the carrier peptide fragment; (2) supplying the construct for transferring a foreign substance into a sample including a target eukaryotic cell; and (3) incubating the sample to which the construct for transferring a foreign substance has been supplied, thereby transferring the construct into the eukaryotic cell in the sample.

## Description

### [Technical Field]

The present invention relates to a method for transferring (carrying) a foreign substance from outside a eukaryotic cell into the cell, and a construct for transferring a foreign substance used in the method. The present application claims priority on the basis of Japanese Patent Application No. 2022-069546 filed on April 20, 2022, and the entire content of this application is incorporated into the specification of the present application by reference.

### [Background Art]

Conventionally, polypeptides and other foreign substances, particularly biologically active substances, have been transferred into the cells of humans and other mammals, etc., (eukaryotic cells) to change the characteristics of the cells (as well as the tissues and organs including the cells) or to improve and enhance the function of the cells.

For example, Patent Literature 1 discloses a construct for transferring a foreign substance, including a foreign substance and a carrier peptide fragment including an amino acid sequence known as a nucleolar localization signal (hereinafter referred to as "NoLS"). Such a construct has cell membrane penetration, so that the foreign substance can be transferred effectively into a target cell. Examples of the carrier peptide fragment disclosed in Patent Literature 1 include NoLS (SEQ ID No.: 2) derived from a basic fibroblast growth factor (FGF2).

### [Citation List]

### [Patent Literature]

### [Patent Literature 1] WO2011/013699

### [Summary of Invention]

Incidentally, in recent years, a technique of transferring a target foreign substance into a cytoplasm with higher efficiency has been demanded from the viewpoints of medical treatment and the like.

The present invention has been made in order to deal with such a demand and it is an object to provide a method for efficiently transferring a target foreign substance from outside a eukaryotic cell into at least a cytoplasm of the cell. It is another object of the present invention to provide a construct for transferring a foreign substance that can efficiently transfer a target foreign substance from outside a eukaryotic cell into at least a cytoplasm of the cell.

In order to create a carrier peptide fragment with higher cell membrane penetration, the present inventors created various modified sequences on the basis of an amino acid sequence with NoLS (SEQ ID No.: 2) derived from FGF2 described above and conducted earnest examinations. As a result, the present inventors found out that the cell membrane penetration is improved by adding an amino acid sequence: tryptophan-proline-cysteine (W-P-C) not containing arginine or lysine, which is generally suggested to contribute to the cell membrane penetration, to a C-terminus of NoLS of FGF2, and thus, completed the present invention.

That is to say, a method disclosed herein is a method for transferring a target foreign substance in vitro from outside a eukaryotic cell into at least a cytoplasm of the cell, and includes the steps of:
(1) preparing a construct for transferring a foreign substance, the construct including a carrier peptide fragment including an amino acid sequence: RSRKYTSWYVALKRWPC (SEQ ID No.: 1), and the target foreign substance that is bonded to an N-terminus and/or a C-terminus of the carrier peptide fragment;
(2) supplying the construct for transferring a foreign substance into a sample including a target eukaryotic cell; and
(3) incubating the sample to which the construct for transferring a foreign substance has been supplied, thereby transferring the construct into the eukaryotic cell in the sample.

The term "foreign substance" used herein encompasses an inorganic or organic compound that is capable of bonding either directly or indirectly via a suitable linker to the N-terminus or C-terminus of the abovementioned carrier peptide fragment, and that has a molecular size and chemical properties that enable the transfer thereof into the eukaryotic cell.

By the method with the above structure, the target foreign substance can be transferred efficiently from outside the eukaryotic cell (outside a cell membrane) into the cytoplasm through the cell membrane.

In one aspect of the method disclosed herein, the foreign substance can be an organic compound selected from the group consisting of a polypeptide, a nucleic acid, a dye, and a drug.

Here, the term "polypeptide" refers to a polymer with a structure in which a plurality of amino acids are joined by peptide bonds. The polypeptide is not limited by the number of peptide bonds (that is, the number of amino acid residues). In other words, the term "polypeptide" encompasses those generally called peptides with about 10 or more and less than 300 amino acid residues and those generally called proteins (typically, high molecular compounds with 300 or more amino acid residues). In this field, polypeptides and proteins are not distinguished strictly. In this specification, polymers formed of a plurality of amino acid residues (including oligomers) are collectively referred to as polypeptides.

Moreover, the term "nucleic acid" refers to a nucleotide polymer and includes DNA and RNA. The term "nucleic acid" is not limited by the number of bases.

In one aspect of the method disclosed herein, the foreign substance can be disposed on the C-terminus of the carrier peptide fragment.

In one aspect of the method disclosed herein, the eukaryotic cell to which the construct for transferring a foreign substance is to be transferred can be a human or nonhuman mammalian cell.

In order to achieve the above object, the present disclosure further provides a construct for transferring a foreign substance artificially manufactured to transfer (carry) a target foreign substance from outside a eukaryotic cell (in particular, various animal cells typified by human and nonhuman mammalian cells that do not have a cell wall) (that is, from outside a cell membrane) into at least a cytoplasm of the cell (preferably, also into a nucleus).

That is to say, the construct for transferring a foreign substance disclosed herein includes: a carrier peptide fragment including an amino acid sequence: RSRKYTSWYVALKRWPC (SEQ ID No.: 1); and the target foreign substance that is bonded to an N-terminus and/or a C-terminus of the carrier peptide fragment.

Such a construct has high cell membrane penetration; thus, it is possible to efficiently transfer a target foreign substance to a target eukaryotic cell.

In one aspect of the construct for transferring a foreign substance disclosed herein, the abovementioned foreign substance can be an organic compound selected from the group consisting of a polypeptide, a nucleic acid, a dye, and a drug.

In one aspect of the construct for transferring a foreign substance disclosed herein, the foreign substance can be disposed on the C-terminus of the carrier peptide fragment.

### [Brief Specification of Drawings]

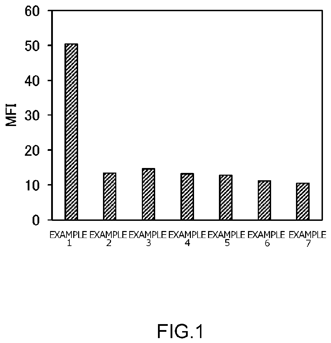

Fig. 1 is a graph expressing values of MFI obtained by culturing with constructs (additives) shown in Examples 1 to 7 added to culture solutions of HeLa cells, and then analyzing the cells with a flow cytometer.

### [Description of Embodiments]

Preferred embodiments of the art disclosed herein will be described below. Matters that are other than matters particularly mentioned in the present specification and that are necessary for the implementation of the art disclosed herein (for example, general matters related to chemical synthesis methods for peptide, cell culture techniques, and preparation of compositions containing peptides or nucleic acids as a component) can be grasped as design matters of those skilled in the art based on the prior art in the fields such as cell engineering, physiology, medical science, pharmacology, organic chemistry, biochemistry, genetic engineering, protein technology, molecular biology, genetics, and the like.

Moreover, the art disclosed herein can be carried out on the basis of the contents disclosed in this specification and common technical knowledge in the fields. In the following specification, the amino acids may be expressed by single letter codes based on the nomenclature for amino acids in the IUPAC-IUB guidelines. Note that the term "amino acid residue" in this specification encompasses N-terminus amino acids and C-terminus amino acids of a peptide chain unless otherwise stated specifically.

In this specification, the term "synthetic peptide" refers to a peptide fragment whose peptide chain alone does not stably exist in nature. The synthetic peptide is manufactured by artificial chemical synthesis or biosynthesis (that is, production based on genetic engineering) and can stably exist in a predetermined composition. Here, the term "peptide" refers to an amino acid polymer having a plurality of peptide bonds and is not limited by the number of amino acid residues.

Note that the amino acid sequence in this specification always expresses the N-terminus on the left side and the C-terminus on the right side.

A construct for transferring a foreign substance disclosed herein includes a carrier peptide fragment including an amino acid sequence: RSRKYTSWYVALKRWPC (SEQ ID No.: 1) and the aforementioned target foreign substance that is bonded to the N-terminus and/or C-terminus of the carrier peptide fragment.

The carrier peptide fragment is a peptide defined (grasped) by the amino acid sequence expressed by SEQ ID No.: 1, and allows the construct to have cell membrane penetration (more preferably, nuclear localization capability (nuclear membrane penetration)) of eukaryotic cells.

The amino acid sequence expressed by SEQ ID No.: 1 corresponds to the sequence obtained by adding tryptophan-proline-cysteine (W-P-C) to the C-terminus of an amino acid sequence expressed by SEQ ID No.: 2. The amino acid sequence expressed by SEQ ID No.: 2 corresponds to the NoLS including 14 amino acid residues in total from 249th to 262nd amino acid residues of FGF2 derived from human. Such an NoLS has been known as having the cell membrane penetration (see Patent Literature 1). The peptide including the amino acid sequence expressed by SEQ ID No.: 1 can exhibit the cell membrane penetration superior to that of the peptide including the amino acid sequence expressed by SEQ ID No.: 2.

The "carrier peptide fragment" disclosed herein is typically the same amino acid sequence as that described above and also encompasses the modified sequence of such an amino acid sequence unless the cell membrane penetration is deteriorated. Here, the "modified sequence" corresponds to an amino acid sequence (modified amino acid sequence) formed by the substitution, deletion, and/or addition (insertion) of one or several (typically, two or three) amino acid residues. Such a slightly modified sequence can be easily used by a person skilled in the art on the basis of the information disclosed herein, and therefore can be encompassed by the term "carrier peptide fragment" as a technical concept disclosed herein.

Typical examples of the modified sequence in this specification include a sequence produced by so-called conservative amino acid replacement in which one, two, or three amino acid residues are conservatively replaced, a sequence in which one, two, or three amino acid residues are added (inserted) to or deleted from a predetermined amino acid sequence, and the like. Typical examples of the conservative amino acid replacement include a sequence in which a basic amino acid residue is replaced by another basic amino acid residue (for example, mutual replacement between a lysine residue and an arginine residue), a sequence in which a hydrophobic amino acid residue is replaced by another hydrophobic amino acid residue (for example, mutual replacement of a leucine residue, an isoleucine residue, and a valine residue), and the like.

The construct for transferring a foreign substance can be designed and configured by bonding (linking), either directly or indirectly via a suitable linker, a desired foreign substance to the N-terminus and/or C-terminus of the abovementioned carrier fragment.

The linker is not limited in particular and may be either a peptidic linker or a non-peptidic linker. Although there is no particular limitation, the amino acid sequence of the peptidic linker is preferably a flexible amino acid sequence that does not cause steric hindrance. Examples of the possible peptidic linker include linkers including ten or less (more preferably one or more and five or less, for example one, two, three, four, or five amino acid residues) amino acid residues including one kind or two or more kinds of amino acid residues selected from glycine, alanine, serine, and the like. As such a linker, β-alanine may be used. As the non-peptidic linker, for example, an alkyl linker, a polyethylene glycol (PEG) linker, an amino hexanoyl spacer, or the like can be used, without particular limitations.

The foreign substance can be, for example, an organic compound such as a polypeptide, a nucleic acid, a dye, or a drug.

If the foreign substance is a polypeptide, the target construct for transferring a foreign substance can be manufactured in such a way that a peptide chain is designed to contain the amino acid sequence constituting the polypeptide and the amino acid sequence constituting the carrier peptide fragment, and then the peptide chain is subjected to biosynthesis or chemical synthesis. Moreover, the construct for transferring a foreign substance can be configured in such a way that an organic compound that acts as a nucleic acid such as various types of DNA or RNA, a dye (for example, various types of fluorescent compounds such as FAM and FITC), or a drug (for example, an antitumor drug including a nucleic acid-based antitumor drug such as 5-fluorouracil (5FU), an antiviral drug such as azidothymidine (AZT), etc.) is directly or indirectly bonded to the N-terminus and/or C-terminus of the above carrier peptide fragment by various publicly known scientific methods.

Although there is no particular limitation, the function of the foreign substance can be, for example, promoting differentiation induction of stem cells (stem cell differentiation inducing activity), growth inhibition of tumor cells (antitumor activity), growth inhibition of virus-infected cells (antivirus activity), or the like.

In the construct for transferring a foreign substance, the number of foreign substances to be bonded to the carrier peptide fragment is not limited in particular. For example, one or more foreign substances may be bonded to one carrier peptide fragment. Although there is no particular limitation, for example, a polypeptide, a nucleic acid, a drug, or the like may be bonded to the C-terminus of one carrier peptide fragment and then a dye may be bonded to the N-terminus thereof. It is preferable to bond the dye to the carrier peptide fragment because it becomes easy to evaluate the efficiency of transferring the construct for transferring a foreign substance into the eukaryotic cell and the localization in the cell.

Note that when the foreign substance is a polypeptide, the polypeptide (amino acid sequence) to be used is not particularly limited. Proteins or a polypeptide with a relatively large number of amino acid residues, for example about 100 to 1000 amino acid residues, can also be used as the foreign substance.

Typically, the total number of amino acid residues constituting the synthetic peptide manufactured as the construct for transferring a foreign substance is several to several tens (for example, 10) or more and suitably 1000 or less, preferably 600 or less, more preferably 500 or less, and particularly preferably 300 or less (for example, 10 to 300). The polypeptide with such a length is easy to synthesize (biosynthesis, chemical synthesis) and easy to use.

Preferably, the foreign substance to be used is a mature form or precursor (including pro-forms and prepro-forms) of a polypeptide involved in a function such as the development, differentiation, growth, malignant transformation, homeostasis, or regulation of metabolism in various cells and tissues (organs). Moreover, the method for transferring a foreign substance disclosed herein can be carried out to transfer a polypeptide with a heretofore unknown function into a cell to elucidate the function of the polypeptide in the cell (in a biological tissue).

For example, if the eukaryotic cell that is the target to which the foreign substance is to be transferred is a human or other mammalian stem cell, it is preferable to use a mature form or precursor of a polypeptide with various biological activities related to the differentiation induction of the stem cells. Note that the term "stem cell" encompasses somatic stem cells, embryonic stem cells, and induced pluripotent stem cells (hereinafter, iPS cells). Moreover, when the eukaryotic cell to which the foreign substance is to be transferred is a cancer cell (tumor cell), it is preferable to use various polypeptides involved in the induction of apoptosis of the cancer cell (tumor cell). Alternatively, in this case, it is preferable to use a polypeptide that can prevent a cancer cell (tumor cell) from inhibiting the function of an immunity monitoring mechanism. Moreover, when the eukaryotic cell that is the target of transfer is a bacteria-infected cell or a virus-infected cell, it is preferable to use various polypeptides involved in inducing apoptosis of these infected cells, a polypeptide that can inhibit the increase of bacteria or virus in the infected cells, or a polypeptide that can inhibit the expansion of the bacteria or virus infection from the infected cells.

Furthermore, similarly to the carrier peptide fragment, the polypeptide as the foreign substance may include a modified amino acid sequence that is formed by the replacement, deletion, and/or addition (insertion) of one or several amino acid residues as long as that function is kept.

In the construct for transferring a foreign substance in which the foreign substance is bonded to the C-terminus of the carrier peptide fragment, an α-amino group of the amino acid residue of the N-terminus of the carrier peptide fragment is preferably acetylated. Although the detailed mechanism is not clear, since the α-amino group of the amino acid of the N-terminus in most of the proteins in the eukaryotic cell is subjected to the acetylation modification, such a structure can improve the stability of the construct in the cells.

In the construct for transferring a foreign substance, the amino acid residue of the C-terminus is preferably amidated. The structural stability (for example, protease resistance) of the construct as described above in the cytoplasm and nucleolus can be improved by amidation of a carboxyl group of the amino acid residue (typically, a C-terminal amino acid residue of a peptide chain). In addition, the amidation of the carboxyl group improves the hydrophilicity of the construct, so that the solubility of this construct in an aqueous solvent can be improved. Examples of such an aqueous solvent include water, various buffer solutions, physiological saline (for example, PBS), a cell culture solution, and the like.

For example, in the case of the construct for transferring a foreign substance in which the foreign substance is bonded to the N-terminus of the carrier peptide fragment, a carboxyl group of cysteine of the C-terminus of the carrier peptide fragment is preferably amidated. In a case where, for example, the foreign substance is a polypeptide and this polypeptide is bonded to the C-terminus of the carrier peptide fragment, it is preferable to amidate the carboxyl group of the C-terminal amino acid residue of the polypeptide.

Among constructs for transferring a foreign substance, those with a relatively short peptide chain (encompassing a polypeptide, a carrier peptide fragment, and a peptidic linker configured as a foreign substance) can easily be manufactured by a general chemical synthesis method. For example, either a conventionally known solid phase or liquid phase synthesis method can be used. A solid phase synthesis method using Boc (t-butyloxycarbonyl) or Fmoc (9-fluoroenylmethoxycarbonyl) as a protecting group for an amino group is preferred. In other words, the aforementioned peptide chain with the desired amino acid sequence and modifications (N-terminal acetylation, C-terminal amidation, etc.) can be synthesized by a solid phase synthesis method using a commercially available peptide synthesizer. Note that only a part of the peptide chain may be synthesized by the aforementioned method and for example, the peptide chain including only the carrier peptide fragment or including the carrier peptide fragment and the peptidic linker can be synthesized.

Alternatively, the peptide part may be manufactured by biosynthesis in accordance with the genetic engineering method. In other words, a polynucleotide (typically, DNA) of a nucleotide sequence (including the ATG start codon) that codes for the desired amino acid sequence is synthesized. Then, a recombinant vector with a genetic construct for expression that includes the synthesized polynucleotide (DNA) and various regulatory elements (including a promoter, ribosome binding site, terminator, enhancer, and various cis-elements for controlling the level of expression) to express the amino acid sequence in a host cell is configured in accordance with the host cell.

Using the general techniques, this recombinant vector is transferred to a predetermined host cell (for example, yeast, insect cell, or plant cell), and the host cell, or an individual or tissue containing the cell is cultured under a predetermined condition. The target peptide can be produced in the cell thereby. Furthermore, the target peptide part can be obtained by isolating the peptide part from the host cell (or from a culture medium if it is secreted) and if necessary, refolding and purifying the peptide part, for example.

Note that the method for configuring the recombinant vector and the method for transferring the configured recombinant vector to the host cell, etc., may utilize methods conventionally used in the fields without modification, and because those methods themselves are not a characterizing feature of the art disclosed herein, the detailed explanation thereof is omitted.

For example, a fusion protein expression system can be used for efficient, large volume production in host cells. More specifically, first, the gene (DNA) coding for the amino acid sequence of the target polypeptide is obtained by chemical synthesis, and the synthesized gene is transferred to a suitable site in a suitable fusion protein expression vector (for example, a glutathione S-transferase (GST) fusion protein expression vector such as the pET series provided by Novagen and the pGEX series provided by Amersham Biosciences). Then, the host cells (typically E. coli) are transformed by the vector. The resulting transformant is cultured to prepare the target fusion protein. Next, the protein is extracted and purified. Then, the resulting purified fusion protein is cleaved by a predetermined enzyme (protease) and the freed target peptide fragment (i.e., the designed artificial polypeptide) is recovered by a method such as affinity chromatography. The target construct for transferring a foreign substance (artificial polypeptide) can be manufactured using this kind of conventionally known fusion protein expression system (for example, the GST/His system provided by Amersham Biosciences can be utilized).

Alternatively, template DNA for use in a cell-free protein synthesis system (i.e., a synthetic gene fragment containing a nucleotide sequence coding for the amino acid sequence of the peptide part for the construct for transferring a foreign substance) can be configured, and in-vitro synthesis of the target polypeptide can be carried out by employing a so-called cell-free protein synthesis system using various compounds necessary for synthesis of the peptide part (ATP, RNA polymerase, amino acids, etc.). References concerning a cell-free protein synthesis system include, for example, the papers by Shimizu et al. (Shimizu et al., Nature Biotechnology, 19, 751-755 (2001)) and Madin et al. (Madin et al., Proc. Natl. Acad. Sci. USA, 97(2), 559-564 (2000)). At the time of the filing of the present application, there are already many companies carrying out polypeptide production on consignment on the basis of the technology disclosed in these documents, and cell-free protein synthesis kits are commercially available (for example, obtainable from Cell Free Sciences Co., Ltd. in Japan).

The nucleotide sequence coding for the peptide part of the construct for transferring a foreign substance and/or a single-chain or double-chain polynucleotide including the nucleotide sequence that is complementary to the above-described nucleotide sequence can be manufactured (synthesized) easily by the conventionally known method. That is to say, by selecting the codon that corresponds to each amino acid residue constituting the designed amino acid sequence, the nucleotide sequence corresponding to such an amino acid sequence is easily determined and provided. Once the nucleotide sequence is determined, the polynucleotide (single chain) corresponding to the desired nucleotide sequence can be easily obtained using a DNA synthesizer or the like. Furthermore, by using the obtained single-chain DNA as a template, the target double-chain DNA can be obtained with various enzymatic synthesizing means (typically, PCR). Moreover, the polynucleotide may be either in a DNA form or an RNA (mRNA, etc.) form. DNA can be provided as a double chain or a single chain. In the case of providing DNA as a single chain, the chain may be either a code chain (sense chain) or a non-code chain (antisense chain) with the sequence complementary to that code chain.

The polynucleotide obtained in this manner can be used as a material for configuring a recombination gene (expression cassette) for peptide production in various host cells or a cell-free protein synthesis system as described above.

The construct for transferring a foreign substance can be suitably used as an effective component of the composition for the usage based on the function of the foreign substance. Note that the construct for transferring a foreign substance may be in a salt form unless the function of the foreign substance is lost. For example, an acid addition salt that can be obtained by carrying out an addition reaction with a normally used inorganic or organic acid in accordance with a conventional method can be used. Thus, the "construct for transferring a foreign substance" according to the present specification and the scope of claims can encompass the construct in such a salt form.

The construct for transferring a foreign substance can be provided not just as the construct for transferring a foreign substance as an effective component but also as a composition that can contain various carriers that are allowable in terms of medical (pharmaceutic) perspectives in accordance with the usage mode.

As the carrier, for example, a carrier that is generally used in the peptide medical fields such as a diluent or an excipient is preferable. As such a carrier, typically, water, a physiological buffer, and various organic solvents are given, although depending as appropriate on the usage or mode of the construct for transferring a foreign substance. Moreover, the carrier can be an alcohol (such as ethanol) aqueous solution with a suitable concentration, glycerol, or non-drying oil such as olive oil, or may be liposome. As a s secondary component that can be contained in a pharmaceutical composition, various filling agents, thickening agents, bonding agents, moisturizers, surfactants, dyes, flavoring agents, and the like are given.

The mode of the composition is not limited in particular. For example, modes of liquids, suspensions, emulsions, aerosols, foams, granules, powders, tablets, capsules, and ointments are given. Moreover, for the use in injection or the like, a lyophilized product or granulated product to prepare a drug solution by being dissolved in physiological saline or a suitable buffer (e.g., PBS), etc., just before use can be formed.

The conventionally known methods may be used for the processes themselves to prepare various modes of medicines (compositions) from the construct for transferring a foreign substance (main component) and various carriers (secondary components) as materials, and a detailed explanation of such a manufacturing process itself is omitted herein because it is not a characterizing feature of the art disclosed herein. For example, Comprehensive Medicinal Chemistry, edited by Corwin Hansch,

Pergamon Press, 1990, can be noted as a source of detailed information concerning formulations.

According to the present disclosure, a method for transferring the construct for transferring a foreign substance in vivo or in vitro using the construct (composition) for transferring a foreign substance disclosed herein is provided. This method, roughly speaking, includes the following steps (1) to (3):
(1) preparing a construct for transferring a foreign substance including a carrier peptide fragment including an amino acid sequence expressed by SEQ ID No.: 1, and a target foreign substance that is bonded to an N-terminus and/or C-terminus of the carrier peptide fragment;
(2) supplying the construct for transferring a foreign substance into a sample including a target eukaryotic cell; and
(3) incubating the sample to which the construct for transferring a foreign substance has been supplied, thereby transferring the construct into the eukaryotic cell in the sample.

The term "eukaryotic cell" encompasses, in vivo, various tissues, viscera, organs, blood, lymph, and the like, for example. The term "eukaryotic cell" encompasses, in vitro, various cell clusters, tissues, viscera, organs, blood, lymph, cell lines, and the like extracted from a body, for example.

The aforementioned composition including the construct disclosed herein can be used in vivo in dosage and method according to the mode and intended purpose. For example, exactly a desired amount of liquid can be administered to a diseased area (for example, malignant tumor tissue, virus-infected tissue, inflamed tissue, etc.) of a patient (i.e., the body) by intravenous, intramuscular, subdermal, intradermal, or intraperitoneal injection. Alternatively, the composition in a solid mode such as a tablet or in a gel-form or aqueous jelly-form such as ointment can be applied directly to a predetermined tissue (i.e., for example, a diseased area such as an organ or a tissue including a tumor cell, virus-infected cell, inflamed cell, or the like). Further alternatively, the composition in a solid mode such as a tablet can be administered orally. In the case of the oral administration, it is preferable to perform encapsulation or apply a protection (coating) material in order to suppress decomposition by digestive enzymes in the alimentary canal.

Alternatively, the composition disclosed herein in an amount suitable for the eukaryotic cell cultured in vitro (that is, a suitable amount of construct for transferring a foreign substance) may be supplied to a culture solution of the target eukaryotic cell at least once. The amount and the number of times per supply are not limited in particular because they can vary depending on the conditions including the kind of eukaryotic cells to culture, the cell density (cell density at the start of culture), the passage number, the culture condition, the kind of culture medium, and the like. For example, the composition is preferably added once, twice, or more times so that the concentration of the carrier peptide fragment in the culture solution falls within the range of about 0.05 µM or more and 100 µM or less, 0.5 µM or more and 50 µM or less, or 1 µM or more and 20 µM or less, for example. In addition, the incubating time after the construct is added is not limited in particular either because the incubating time can vary depending on the kind of eukaryotic cells and various conditions. For example, the incubating time can be 0.5 hours or more, 1 hour or more, 4 hours or more, 8 hours or more, or 20 hours or more. Moreover, the incubating condition is not limited in particular because the incubating condition can vary depending on the kind of eukaryotic cells; however, the incubation is possible in a 5% CO₂ atmosphere under 37°C, for example.

Note that one example of the transferring method in vitro will be described in a test example below.

A method for evaluating the transferring efficiency of the construct for transferring a foreign substance is not limited in particular. For example, in the case where a dye (typically, a fluorescent compound) is bonded to the construct, it is possible to evaluate the transferring efficiency into the eukaryotic cells by using microscope observation (for example, fluorescence microscope observation), flow cytometry, or the like. Alternatively, the transferring efficiency of the construct can be evaluated by an immunochemical method (for example, Western Blot, immunocytochemistry, or the like) that uses an antibody to recognize the peptide part of the construct specifically.

As described above, the following items are given as specific aspects of the art disclosed herein.
Item 1: The method for transferring a target foreign substance in vitro from outside the eukaryotic cell into at least the cytoplasm of the cell, the method including the steps of: (1) preparing the construct for transferring a foreign substance, the construct including the carrier peptide fragment including the amino acid sequence: RSRKYTSWYVALKRWPC (SEQ ID No.: 1), and the target foreign substance that is bonded to the N-terminus and/or the C-terminus of the carrier peptide fragment; (2) supplying the construct for transferring a foreign substance into the sample including the target eukaryotic cell; and (3) incubating the sample to which the construct for transferring a foreign substance has been supplied, thereby transferring the construct into the eukaryotic cell in the sample.
Item 2: The method according to Item 1, in which the foreign substance is the organic compound selected from the group consisting of a polypeptide, a nucleic acid, a dye, and a drug.
Item 3: The method according to Item 1 or 2, in which the foreign substance is disposed on the C-terminus of the carrier peptide fragment.
Item 4: The method according to any one of Items 1 to 3, in which the eukaryotic cell to which the construct for transferring a foreign substance is to be transferred is the human or nonhuman mammalian cell.
Item 5: The construct for transferring a foreign substance that is manufactured in order to transfer the target foreign substance from outside the eukaryotic cell into at least the cytoplasm of the cell, the construct including: the carrier peptide fragment including the amino acid sequence: RSRKYTSWYVALKRWPC (SEQ ID No.: 1); and the target foreign substance that is bonded to the N-terminus and/or the C-terminus of the carrier peptide fragment.
Item 6: The construct according to Item 5, in which the foreign substance is the organic compound selected from the group consisting of a polypeptide, a nucleic acid, a dye, and a drug.
Item 7: The construct according to Item 5 or 6, in which the foreign substance is disposed on the C-terminus of the carrier peptide fragment.

Several test examples concerning the art disclosed herein are described below, but it is not intended to limit the art disclosed herein to these test examples.

### <Manufacture of construct for transferring foreign substance>

Synthetic peptides (peptides 1 to 6) with amino acid sequences shown in Table 1 were prepared. The amino acid sequences expressed by SEQ ID Nos.: 3 to 6 are the sequences in which one or two arginine residues in the amino acid sequence of the NoLS in FGF2 expressed by SEQ ID No.: 2 are replaced by a lysine residue. The peptides 1 to 6 were synthesized by carrying out a solid phase synthesis method (Fmoc method) in accordance with the manual using a commercially available peptide synthesizer. In the synthesized peptides 1 to 6, the α-amino group of the amino acid residue of the N-terminus is acetylated. Note that a detailed explanation of the mode of usage of the peptide synthesizer itself is omitted because it is not a characterizing feature of the art disclosed herein.

### [Table 1]

**Table 1**

| Peptide No. | Sequence | SEQ ID No. |
|---|---|---|
| 1 | RSRKYTSWYVALKRWPC | 1 |
| 2 | RSRKYTSWYVALKR | 2 |
| 3 | KSKKYTSWYVALKR | 3 |
| 4 | KSRKYTSWYVALKR | 4 |
| 5 | RSKKYTSWYVALKR | 5 |
| 6 | RSRKYTSWYVALKK | 6 |

Next, FAM (C₂₁H₁₂O₇: 5(6)-carboxyfluorescein, molecular weight 376.3, excitation wavelength 495 nm, fluorescence wavelength 520 nm) corresponding to fluorescent dye was bonded as the foreign substance directly to the amino acid residue of the C-terminus of each of the peptides 1 to 6 in accordance with a conventional method. Thus, a construct including the peptide 1 (also referred to as "sample 1"), a construct including the peptide 2 (also referred to as "sample 2"), a construct including the peptide 3 (also referred to as "sample 3"), a construct including the peptide 4 (also referred to as "sample 4"), a construct including the peptide 5 (also referred to as "sample 5"), and a construct including the peptide 6 (also referred to as "sample 6") were obtained. The samples 1 to 6 were each diluted in dimethyl sulfoxide (DMSO) to prepare sample solutions 1 to 6 with a sample concentration of 2 mM.

### <Evaluation of cell membrane penetration by flow cytometry>

As the eukaryotic cells, HeLa cells (established cell strain derived from a human cervical cancer cell) were used and the cell membrane penetration of the peptides 1 to 6 was analyzed. In Examples 1 to 6, the prepared samples 1 to 6 were used, respectively, and in Example 7, the FAM solution was used.

### (Example 1)

The HeLa cell was cultured in 10% fetal bovine serum (FBS)-containing Dulbecco's modified Eagle's medium (DMEM (Cat No.043-30085, manufactured by FUJIFILM Wako Pure Chemical Corporation)), which is a normal culture medium.

The HeLa cell that adhered to a culture plate was cleaned with PBS and then, a 0.25%-trypsin/EDTA solution was added thereto, and incubation was performed for three minutes at 37°C. After the incubation, the aforementioned 10% FBS-containing DMEM was added to deactivate trypsin and then, five-minute centrifugal separation was performed at 150 × g to precipitate the cells. After the supernatant generated by the centrifugal separation was removed, the 10% FBS-containing DMEM was added to the precipitate (cell pellet) to prepare a cell suspension of about 2 × 10⁵ cells/mL. The cell suspension was added by 1 mL to a well of a commercially available six-hole (well) plate (manufactured by AGC TECHNO GLASS Co., Ltd.) so as to seed the cell (about 2 × 10⁵ cells/well). In addition, the 2 mM sample solution 1 was diluted with the 10% FBS-containing DMEM to prepare the sample solution 1 in which the concentration of the sample 1 was 20 µM. Then, 1 mL of the 20 µM sample solution 1 was added to the well (that is, so that the concentration of the sample 1 became 10 µM and the DMSO concentration became 0.5% in the culture solution in the well). After that, the cell was incubated for 20 hours at 37°C under a 5% CO₂ condition.

After the 20-hour incubation, the culture supernatant was removed from the well and the cells in the well were cleaned twice with 1 mL of PBS. Next, 200 µL of the 0.25% trypsin/EDTA solution was added to the well and the incubation was performed for three minutes at 37°C. After the incubation, 400 µL of the 10% FBS-containing DMEM was added to the well to deactivate the trypsin. Subsequently, the cell suspension in the well was transferred to a tube and the cells were collected. After that, 600 µL of PBS was added further to the well to clean the well. Then, PBS in the well was transferred to the tube, so that the cells remaining in the well were collected in the tube. This tube was subjected to five-minute centrifugal separation at 4°C under a condition of 210 × g. After the centrifugal separation, the supernatant was removed and the precipitate (cell pellet) was suspended (cleaned) with 1 mL of PBS, which was followed by the centrifugal separation under the same condition as that described above. After this operation was repeated twice, the supernatant was removed and thus, the cells (cell pellet) cultured in a culture medium containing the sample 1 were obtained.

Regarding the obtained cells (cell pellet), the cell membrane penetration of the sample 1 was analyzed using a flow cytometer. As the flow cytometer, On-Chip Flowcytometer (manufactured by On-Chip Biotechnologies Co., LTD.) was used.

For such an analysis, the obtained cell pellet was suspended with 50 µL of PBS. To this suspension, additionally, 50 µL of 2 × sample buffer for the flow cytometer was added so as to prepare a cell suspension for analysis.

Using the aforementioned flow cytometer, gating based on forward scatter (FSC) and side scatter (SSC) was performed to set a gate about a cell group to be analyzed, and the fluorescence intensity was measured about the cell group in this gate. Note that the analysis was performed so that the cell group contained at least 10000 or more cells. The fluorescence intensity was measured using a fluorescence detector FL2 of the flow cytometer (optimum detection wavelength around 543 nm) capable of detecting the fluorescence wavelength of FAM. Regarding this measurement result, the analysis was performed using commercially available analysis software "FlowJo (registered trademark)" (manufactured by TreeStar) to obtain mean fluorescence intensity (MFI) of the cell group to be measured.

### (Example 2)

The process similar to that in Example 1 was performed except that the prepared sample solution 2 was used instead of the sample solution 1.

### (Example 3)

The process similar to that in Example 1 was performed except that the prepared sample solution 3 was used instead of the sample solution 1.

### (Example 4)

The process similar to that in Example 1 was performed except that the prepared sample solution 4 was used instead of the sample solution 1.

### (Example 5)

The process similar to that in Example 1 was performed except that the prepared sample solution 5 was used instead of the sample solution 1.

### (Example 6)

The process similar to that in Example 1 was performed except that the prepared sample solution 6 was used instead of the sample solution 1.

### (Example 7)

The process similar to that in Example 1 was performed except that the FAM solution diluted with DMSO was used instead of the sample solution 1. The concentration of this FAM solution was the same as the concentration of the sample 1 solution (that is, the FAM concentration was 10 µM and the DMSO concentration was 0.5% in the culture solution in the well).

The results obtained from Examples 1 to 7 are shown in Table 2 and Fig. 1. Fig. 1 is a graph expressing the values of MFI in each example.

### [Table 2]

**Table 2**

| | Structure of construct (additive) | MFI |
|---|---|---|
| Example 1 | Ac-RSRKYTSWYVALKRWPC-FAM | 50.4 |
| Example 2 | Ac-RSRKYTSWYVALKR-FAM | 13.4 |
| Example 3 | Ac-KSKKYTSWYVALKR-FAM | 14.6 |
| Example 4 | Ac-KSRKYTSWYVALKR-FAM | 13.2 |
| Example 5 | Ac-RSKKYTSWYVALKR-FAM | 12.8 |
| Example 6 | Ac-RSRKYTSWYVALKK-FAM | 11.1 |
| Example 7 | FAM | 10.5 |

As shown in Table 2 and Fig. 1, the value of MFI was remarkably higher in Example 1 than in Example 2. That is to say, it has been confirmed that the construct including the peptide 1 exhibits superior cell membrane penetration to the construct including the peptide 2 that is known to have the cell membrane penetration. In addition, it is understood that the cell membrane penetration is not largely different in Examples 3 to 6 in which one or two arginine residues of the peptide 2 are replaced by a lysine residue.

Although the detailed data is not shown, the present inventors' examination indicates that the construct including the peptide 1 enables the foreign substance, which is not just a fluorescent dye but also a polypeptide, a nucleic acid, or a drug, to be transferred from outside the cell into the cytoplasm efficiently.

As is clear from the above specification, it is understood that the construct for transferring a foreign substance disclosed herein has the excellent cell membrane penetration and thus, the target foreign substance can be transferred efficiently from outside the eukaryotic cell into at least the cytoplasm of the cell.

The specific examples of the art disclosed herein have been described above in detail; however, these are just examples and will not limit the scope of claims. The art described in the scope of claims includes those in which the specific examples given above are variously modified and changed.

The art disclosed herein provides the artificially manufactured construct for transferring a target foreign substance from outside the eukaryotic cell (in particular, various animal cells typified by human and nonhuman mammalian cells that do not have a cell wall) into the cytoplasm thereof. By utilizing this construct, the target foreign substance can be effectively transferred into the target cell, and biological tissues such as organs including cells to which the foreign substance has been transferred and cells that contain the foreign substance can be obtained. In addition, by utilizing this construct, therapeutic agents for diseases can be provided.

## Claims

1. A method for transferring a target foreign substance in vitro from outside a eukaryotic cell into at least a cytoplasm of the cell, the method comprising the steps of:
(1) preparing a construct for transferring a foreign substance, the construct including
a carrier peptide fragment including an amino acid sequence: RSRKYTSWYVALKRWPC (SEQ ID No.: 1), and
the target foreign substance that is bonded to an N-terminus and/or a C-terminus of the carrier peptide fragment;
(2) supplying the construct for transferring a foreign substance into a sample including a target eukaryotic cell; and
(3) incubating the sample to which the construct for transferring a foreign substance has been supplied, thereby transferring the construct into the eukaryotic cell in the sample.

2. The method according to claim 1, wherein the foreign substance is an organic compound selected from the group consisting of a polypeptide, a nucleic acid, a dye, and a drug.

3. The method according to claim 1 or 2, wherein the foreign substance is disposed on the C-terminus of the carrier peptide fragment.

4. The method according to claim 1 or 2, wherein the eukaryotic cell to which the construct for transferring a foreign substance is to be transferred is a human or nonhuman mammalian cell.

5. A construct for transferring a foreign substance that is manufactured in order to transfer a target foreign substance from outside a eukaryotic cell into at least a cytoplasm of the cell, the construct comprising:
a carrier peptide fragment including an amino acid sequence: RSRKYTSWYVALKRWPC (SEQ ID No.: 1); and
the target foreign substance that is bonded to an N-terminus and/or a C-terminus of the carrier peptide fragment.

6. The construct according to claim 5, wherein the foreign substance is an organic compound selected from the group consisting of a polypeptide, a nucleic acid, a dye, and a drug.

7. The construct according to claim 5 or 6, wherein the foreign substance is disposed on the C-terminus of the carrier peptide fragment.
